Europäisches Patentamt

European Patent Office

Office européen des brevets

(11) Publication number: **0 192 660 B1**

# EUROPEAN PATENT SPECIFICATION

(45) Date of publication of patent specification: **19.11.92**

(51) Int. Cl.⁵: **A61K 37/04**, A61K 37/48, A61K 31/66, A61K 31/52, A61K 31/155, A61K 33/02, A61K 31/505, A61K 31/17, A61K 31/16, A61K 31/13

(21) Application number: **85903967.9**

(22) Date of filing: **08.08.85**

(86) International application number:
**PCT/US85/01505**

(87) International publication number:
**WO 86/01110 (27.02.86 86/05)**

(54) **ABSORPTION ENHANCING AGENTS.**

(30) Priority: **08.08.84 US 638695**
**06.03.85 US 708845**
**27.03.85 US 716705**
**20.05.85 US 735734**

(43) Date of publication of application:
**03.09.86 Bulletin 86/36**

(45) Publication of the grant of the patent:
**19.11.92 Bulletin 92/47**

(84) Designated Contracting States:
**AT BE CH DE FR GB IT LI LU NL SE**

(56) References cited:
**US-A- 3 629 410**
**US-A- 4 292 311**
**US-A- 4 346 709**

**Chemical Abstracts, Vol. 97. Published 1982, 21610S, Lankin et al**

**Chemical Abstracts, Vol. 98, Published 1983, 46932G, Mil'chakov**

(73) Proprietor: **SURVIVAL TECHNOLOGY, INC.**
**8101 Glenbrook Road**
**Bethesda, MD 20814(US)**

(72) Inventor: **SARNOFF, Stanley, Jay**
**7507 Hampden Lane**
**Bethesda, MD 20814(US)**

(74) Representative: **UEXKÜLL & STOLBERG Patentanwälte**
**Beselerstrasse 4**
**W-2000 Hamburg 52(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Reference is made to Applicant's co-pending application EP-A-0191056 and Application EP-A-0214281 which disclose the use of a t-PA absorption enhancing agent, e.g. hydroxylamine or a non-toxic salt thereof, preferably hydroxylamine hydrochloride, to increase the absorption of t-PA in the blood when the t-PA is administered intramuscularly.

Superoxide dismutase (SOD) is a protein, specifically an enzyme, which has been used to ameliorate the side effects of radiation therapy, more especially to protect the healthy cells.

Superoxide dismutase has also been employed to scavenge oxygen free radicals generated at the time of reperfusion and thus to diminish myocardium damage. Oxygen derived free radicals include the superoxide radical $O_2-$ and the more reactive hydroxyl radical OH which may be produced secondarily from superoxide.

In the JACS Volume 5, No. 2, February 1985 there are Abstracts of a number of articles on this subject. Thus there is an Abstract of an article by Ambrosio et al entitled "Reduction In Reperfusion Injury With Recombinant Human Superoxide Dismutase Following Global Ischemia" on page 489 and also on page 489 an Abstract of an article by Werns et al entitled "Superoxide Dimustase But Not Catalase Protects Reperfused Ischemic Myocardium." Also there is an Abstract of an article by Horneffer et al entitled "Reperfusion Injury Prevented By Oxygen Free Radical Scavenger: Functional and Morphological Evidence" on page 541 and an Abstract of an article by Myers et al entitled "Superoxide Dismutase And Catalase Enhance Recovery Of Myocardial Function Following Reversible Ischemia" on page 542. The Horneffer et al article points out that with reperfusion of ischemic myocardium, there is production of cytotoxic oxygen free-radicals. Horneffer et al tested SOD to see whether it might improve the salvage of ischemic myocardium. It was found that infusion of SOD in pigs after reversible coronary occlusion prevented reperfusion injury and improved myocardial salvage. Similar results were obtained by Myers et al on dogs using 5 mg/kg (3100 U/mg) of SOD. Ambrosio et al found that recombinant human superoxide dismutase (SOD) reduced reperfusion injury.

Recently McCord, New England Journal of Medicine, Volume 312, No. 3, pages 159-163 pointed out that "ischemic injury" in many cases is a misnomer and that a substantial part of the injury may be more properly called reperfusion injury and occurs during the period when molecular oxygen is reintroduced into the tissue. It was also noted by McCord that the primary source of superoxide in reperfused reoxygenated tissues appears to be the enzyme xanthine oxidase released during ischemia by a calcium-triggered proteolytic attack on xanthine dehydrogenase. Reperfused tissues can be protected by scavengers of superoxide radicals or hydroxyl radials or by allopurinol or other inhibitors of xanthine oxidase, e.g. alloxanthine.

The compound WR-2721 [$H_2N(CH_2)_3$ $NHCH_2CH_2SPO_3H_2$] has been used to protect against the cytotoxic effects of irradiation. WR-2721 also protects the healthy cells when treating cancer with chemotherapy.

It has now been discovered that WR-2721 can be used in place of SOD to scavenge oxygen free radicals generated at the time of reperfusion. The WR-2721 can be administered either intravenously or intramuscularly in the same manner as SOD and in the same dosage.

Additionally it has now been found there can be obtained more rapid and prolonged reperfusion and thrombolysis by the intramuscular administration of a scavenger of oxygen free radicals or hydroxyl groups, e.g. superoxide dismutase or other proteins, e.g. enzymes which act as scavengers or WR-2721 or analogous materials together with an agent enhancing the absorption of the scavenger. Likewise, there can be administered intramuscularly inhibitors of xanthine oxidase such as allopyrinol and alloxanthine.

The present invention relates to a package containing (1) a scavenger of oxygen free radicals generated at the time of reperfusion in a mammal or an agent which prevents cytotoxic effects on healthy cells when a mammal is subjected to irradiation or chemotherapy and (2) a substance enhancing the absorption of said scavenger or agent, selected from urea, substituted ureas, hydantoin, 5-substituted hydantoins, 5,5-dialkylhydantoins, 5,5-diarylhydantoins, guanidine, methylguanidine, hydrazine, alkyl hydrazines, aryl hydrazines, hydroxylamine, alkyl hydroxylamines, aryl hydroxyamines and non-toxic salts thereof.

Furthermore, the intramuscular administration of SOD or WR-2721 and related materials can be employed when these agents are employed as protective agents for healthy cells in treating cancer by irradiation or chemotherapy.

The intramuscular administration of the present invention is of importance where there is a need for a fast response time and it cannot be obtained by intravenous injection.

In place of WR-2721 there can also be employed analogous materials of the formula

$$H_2N\text{-}R(NHR_2)_x\text{-}NHR_3\text{-}SPO_3H_2 \quad (I)$$

where $R_1$, $R_2$, and $R_3$ are each alkylene of at least two carbon atoms, e.g. 2 to 6 carbon atoms and x is zero or a small integer, e.g. 1 to 3. WR-2721 is the compound within formula I which is S-aminotrimethyleneaminoethyl thiophosphate. Other compounds within this formula are S-aminoethyleneaminoethyl thiophosphate, S-aminoethyleneaminopropyl thiophosphate, S-amineothyleneaminoethyleneaminoethyl thiophosphate.

The invention includes packaging the scavenger, xanthine oxidase inhibitor or cell protective agent with the agent enhancing the absorption of the scavenger, inhibitor or cell protective agent in the blood. The enhancing agent preferably is hydroxylamine hydrochloride. There can be used, for example, a known emergency type automatic injector and the process comprises injecting the two medicament agents into the muscle tissue.

In accordance with the principles of the present invention, the absorption rate of SOD, WR-2721 and other scavengers and protective agents in the blood is enhanced by utilizing with the SOD, WR-2721 or other scavenger or protective agent dosage, a dosage of an absorption enhancing agent for SOD, WR-2721 or the like, preferably hydroxylamine hydrochloride. Preferably, the absorption enhancing agent such as hydroxylamine hydrochloride is mixed in with the SOD, WR-2721 or other scavenger or protective agent dosage to form a single mixed dosage which is then injected intramuscularly (i.m.). It is within the contemplation of the present invention to inject the absorption enhancing agent as a separate dosage within the same site as the separate dosage of SOD, WR-2721 or other scavenger or protective agent (e.g. U.S. patent 4,394,863). An example of an amount of absorption enhancing agent, such as hydroxylamine hydrochloride, which is added to the SOD, WR-2721 or other scavenger or protective agent dosage, as previously described, to form a single mixed dosage is an amount of from 0.25 to 100 milligrams per kilogram of body weight. For example with dogs using a dosage of 5 mg/kg of SOD there can be used 5 mg/kg of hydroxylamine hydrochloride.

As the absorption enhancing agent hydroxylamine is preferably employed in the form of a non-toxic water soluble salt. Thus there can be used, for example, in place of hydroxylamine salts such as hydroxylamine hydrochloride, hydroxylamine hydrobromide, hydroxylamine hydroiodide, hydroxylamine sulfate, hydroxylamine nitrate, hydroxylamine acetate, and hydroxylamine propionate. Most preferably there is employed hydroxylamine hydrochloride.

The absorption enhancing agents in accordance with the invention are selected from urea, mono and dialkyl ureas, e.g. methyl urea, ethyl urea, propyl urea, butyl urea, N,N-dimethyl urea, N,N-diethyl urea, N,N-diisopropyl urea, mono and dialkyl ureas, e.g. phenyl urea, p-tolylurea, N,N-diphenyl and urea, N,N-di-p-tolyl urea, thiourea, hydantoin, 5-substituted hydantoins, e.g. 5-alkyl, 5-aralkyl, and 5-aryl hydantoins and 5,5-dialkyl and 5,5-diaryl hydantoins, e.g. 5-methyl hydantoin, 5-ethyl hydantoin, 5,5-dimethyl hydantoin, 1,5-trimethylene hydantoin, 1,5-tetramethylene hydantoin, 5-phenyl hydantoin, 5-p-tolyl-hydantoin, and 5,5-diphenyl hydantoin, guanidine, methyl guanidine, hydrazine, alkyl and aryl hydrazines, e.g. methyl hydrazine, ethyl hydrazine, butyl hydrazine, phenyl hydrazine and diphenyl hydrazine, alkyl and aryl hydroxylamines, e.g. methyl hydroxylamine, ethyl hydroxylamine and phenyl hydroxylamine. The substituted ureas, hydrazines and hydroxylamines likewise can be used in the form of salts, e.g. as hydrochlorides.

The simultaneous administration of SOD, WR-2721 or other scavenger, inhibitor or protective agent and absorption enhancing agent is intended for not only human use but it is within the scope of the invention that they be administered for veterinary purposes, e.g. to other mammals, e.g., dogs, cat, cattle, rabbits, and horses.

While it is not necessary to employ catalase with the SOD there can be simultaneously employed catalase, e.g. to possibly further prolong the activity of SOD as mentioned by Werns et al.

In another aspect the invention includes a method of treating a mammal, e.g. any of those mentioned previously with a liquid medicament under circumstances where intravenous injection is not practical but the fast response time of an intravenous injections is desirable. This is accomplished by administering in liquid form the medicament together with hydroxylamine or a non-toxic salt thereof, e.g. any of those mentioned above intramuscularly. There can be used for example, either a single mixed dosage or separate dosages in the manner set forth above.

The hydroxylamine or salt thereof can be used in an amount of from 0.25 to 100 milligrams per kilogram of body weight. Thus, for example, to treat hyperglycemia there can be injected intramuscularly to a 68kg (150 pound) individual 1-2 mg of glucagon and 350 mg of hydroxylamine hydrochloride. Also there can be injected intramuscularly to a 68kg (150 pound) individual in need of lidocaine 300 mg of lidocane and 350 mg of hydroxylamine hydrochloride. With insulin for example, 21 units of regular insulin can be injected together with 350 mg of hydroxylamine hydrochloride.

The allopurinol or alloxanthine can be administered intramuscularly, e.g. in an amount of 1-1000 mg, e.g. 250 mg, without hydroxylamine or salt thereof. However, to enhance the absorption of the xanthine oxidase inhibitor it is preferred to add the hydroxyl amine or salt thereof. Thus, there can be injected intramuscularly 100 mg of allopurine together with 350 mg of hydroxylamine hydrochloride.

**Claims**

1. A package containing (1) a scavenger of oxygen free radicals generated at the time of reperfusion in a mammal or an agent which prevents cytotoxic effects on healthy cells when a mammal is subjected to irradiation or chemotherapy and (2) a substance enhancing the absorption of said scavenger or agent, selected from urea, substituted ureas, hydantoin, 5-substituted hydantoins, 5,5-dialkylhydantoins, 5,5-diarylhydantoins, guanidine, methylguanidine, hydrazine, alkyl hydrazines, aryl hydrazines, hydroxylamine, alkyl hydroxylamines, aryl hydroxyamines and non-toxic salts thereof.

2. A package according to claim 1 wherein the substance enhancing the absorption is hydroxylamine or a non-toxic salt thereof.

3. A package according to claim 2, wherein the non-toxic salt of hydroxylamine is hydroxylamine hydrochloride.

4. A package according to claims 1 to 3, wherein (1) and (2) are each in a separate dosage form.

5. A package according to claims 1 to 3, wherein (1) and (2) are mixed within a single dosage form.

6. A package according to claims 1 to 5, wherein (1) is super-oxide dismutase.

7. A package according to claims 1 to 5, wherein (1) is S-aminotrimethyleneaminoethyl thiophosphate.

8. A package according to claims 1 to 5, wherein (1) is an inhibitor of xanthine oxidase.

9. A package according to claim 8, wherein (1) is allopurinol.

10. A package according to claim 8, wherein (1) is alloxanthine.

11. Use of hydroxylamine or a non-toxic salt thereof for the preparation of a medicament for intramuscular administration of a scavenger of oxygen free radicals generated at the time of reperfusion or an agent which prevents cytotoxic effects on healthy cells when a mammal is subjected to irradiation or chemotherapy and for increasing the absorption of the scavenger or agent into the blood.

**Patentansprüche**

1. Packung, die (1) eine Fängersubstanz für sauerstofffreie Radikale, welche sich bei einer Reperfusion in einem Säugetier gebildet haben, oder ein Mittel, welches gesunde Zellen vor cytotoxischen Effekten schützt, wenn ein Säugetier einer Bestrahlung oder Chemotherapie ausgesetzt wird, und (2) eine die Absorption der Fängersubstanz oder des Mittels verbessernde Substanz enthält, die aus Harnstoff, substituierten Harnstoffen, Hydantoin, 5-substituierten Hydantoinen, 5,5-Dialkylhydantoinen, 5,5-Diarylhydantoinen, Guanidin, Methylguanidin, Hydrazin, Alkylhydrazinen, Arylhydrazinen, Hydroxylamin, Alkylhydroxylaminen, Arylhydroxylaminen und nichttoxischen Salzen davon ausgewählt ist.

2. Packung nach Anspruch 1, in der die die Absorption verbessernde Substanz Hydroxylamin oder ein nicht-toxisches Salz davon ist.

3. Packung nach Anspruch 2, in der das nicht-toxische Salz von Hydroxylamin Hydroxylaminhydrochlorid ist.

4. Packung nach den Ansprüchen 1 bis 3, in der (1) und (2) jeweils in einer separaten Dosierungsform vorliegen.

5. Packung nach den Ansprüchen 1 bis 3, in der (1) und (2) innerhalb einer einzigen Dosierungsform vermischt vorliegen.

6. Packung nach den Ansprüchen 1 bis 5, in der (1) Superoxiddismutase ist.

7. Packung nach den Ansprüchen 1 bis 5, in der (1) S-Aminotrimethylenaminoethylthiophosphat ist.

8. Packung nach den Ansprüchen 1 bis 5, in der (1) ein Inhibitor der Xanthinoxidase ist.

9. Packung nach Anspruch 8, in der (1) Allopurinol ist.

10. Packung nach Anspruch 8, in der (1) Alloxanthin ist.

11. Verwendung von Hydroxylamin oder einem nicht-toxischen Salz davon zur Herstellung eines Medikaments zur intramuskulären Verabreichung einer Fängersubstanz für sauerstofffreie Radikale, welche sich bei einer Reperfusion gebildet haben, oder eines Mittels, welches gesunde Zellen vor cytotoxischen Effekten schützt, wenn ein Säugetier einer Bestrahlung oder Chemotherapie ausgesetzt wird, und zur Verbesserung der Absorption der Fängersubstanz oder des Mittels in das Blut.

**Revendications**

1. Emballage contenant (1) un fixateur de radicaux libres pourvus d'oxygène, engendrés au moment de la reperfusion dans un mammifère, ou un agent qui prévient les effets cytotoxiques sur des cellules saines lors-qu'un mammifère est soumis à une chimiothérapie ou une irradiation et (2) une substance qui augmente l'absorption du fixateur ou de l'agent précité, choisie parmi l'urée, les urées substituées, l'hydantoïne, les hydantoïnes 5-substituées, les 5,5-dialkylhydantoïnes, les 5,5-diarylhydantoïnes, la guanidine, la methylguanidine, l'hydrazine, des alkylhydrazines, les arylhydrazines, l'hydroxylamine, les alkylhydroxylamines, les arylhydroxylamines et leurs sels atoxiques.

2. Emballage suivant la revendication 1, caractérisé en ce que la substance qui augmente l'absorption est l'hydroxylamine ou un sel atoxique de celle-ci.

3. Emballage suivant la revendication 2, caractérisé en ce que le sel atoxique de l'hydroxylamine est le chlorhydrate d'hydroxylamine.

4. Emballage suivant les revendications 1 à 3, caractérisé en ce que (1) et (2) se présentent chacun sous une forme de dosage séparée.

5. Emballage suivant les revendications 1 à 3, caractérisé en ce que (1) et (2) sont mélangés en une forme de dosage unique.

6. Emballage suivant les revendications 1 à 5, caractérisé en ce que (1) est la superoxydedismutase.

7. Emballage suivant les revendications 1 à 5, caractérisé en ce que (1) est le thiophosphate de S-aminotriméthylèneaminoéthyle.

8. Emballage suivant les revendications 1 à 5, caractérisé en ce que (1) est un inhibiteur de la xanthineoxydase.

9. Emballage suivant la revendication 8, caractérisé en ce que (1) est l'allopurinol.

10. Emballage suivant la revendication 8, caractérisé en ce que (1) est l'alloxanthine.

11. Utilisation de l'hydroxylamine ou d'un sel atoxique de celle-ci en vue de la préparation d'un médicament destiné à l'administration par la voie intramusculaire d'un fixateur de radicaux libres pourvus d'oxygène, engendrés au moment de la reperfusion, ou d'un agent qui prévient les effets cytotoxiques sur des cellules saines lorsqu'un mammifère est soumis à une chimiothérapie ou une irradiation et en vue d'augmenter l'absorption du fixateur ou de l'agent dans le sang.